# EUROPEAN PATENT APPLICATION

(11) **EP 4 467 002 A1**
(43) Date of publication of application: **27.11.2024**
(21) Application number: 22920097.7
(22) Date of filing: 30.12.2022
(51) Int. Cl.: A23J 1/12

(54) **METHOD FOR PREPARING STARCH AND NON-THERMAL-DENATURATION PROTEIN POWDER BY USING RICE AS RAW MATERIAL**

(30) Priority: 17.01.2022 CN 202210047112
(71) Applicant: Zou, Jiarui, Wuhan, Hubei 430023 (CN)
(72) Inventor: ZOU, Chuanjun, Wuhan, Hubei 430023 (CN)
(74) Representative: Zeuner Summerer Stütz
(86) International application number: PCT/CN2022/143708
(87) International publication number: WO 2023/134467

(57) **Abstract**

Provided is a method for preparing starch and a non-thermal-denaturation protein powder by using rice as a raw material. The method comprises: crushing, pulping, homogenizing and centrifugally removing water from rice to obtain a solid phase and wastewater; and adding a specific gravity regulator, i.e. a syrup, into the solid phase, and separating the material into three phases: a heavy phase, a light phase and a liquid phase, wherein the heavy phase is used for preparing starch, the light phase is used for preparing a protein powder and a syrup, part of the liquid phase is reused, and the other part of the liquid phase is used for preparing the syrup; the wastewater generated during the preparation process is reused for the preparation of a syrup material; and the syrup is used as the specific gravity regulator.

## Description

### TECHNICAL FIELD

The present application relates to the technical field of deep processing of rice, and more particularly relates to a method for preparing starch and a non-thermal-denaturation protein powder by using rice as a raw material.

### BACKGROUND

Vegetable protein powder refers to a powdered substance formed after crushing, purification, drying and other processing of proteins extracted from plants such as grains and beans.

Compared with other plant starch granules, rice starch granules are very small, 2-8 µm in size, and uniform in particle size. Simultaneously producing starch and protein powder by using rice as a raw material mainly adopts alkali-solution and acid-isolation. Rice protein is mainly alkalisoluble gluten. The protein is solubilized with an alkali and then filtered to obtain starch. The solubilized protein is then acidified to obtain a protein precipitate. The protein requires high temperature sterilization and spray drying. This is the most mature method at present, and is also a method actually used in production.

Other methods also include an enzymatic method in which protein is hydrolyzed with an enzyme to obtain starch or starch is hydrolyzed with an enzyme to obtain protein. Other methods also include solvent extraction, physical separation, complex extraction and the like.

The defects in the prior art are as follows:
the alkali-solution and acid-isolation requires the consumption of a large amount of alkali and generates a large amount of wastewater, the alkali destroys the structures of protein and starch, many harmful side reactions are generated, and high temperature sterilization and spray drying before protein drying will also cause protein denaturation.

The enzymatic method is relatively mild in process, but the production efficiency is low, and starch and protein cannot be obtained simultaneously.

A solvent method has low efficiency and complex process, making it unsuitable for industrialization.

Therefore, it is necessary to provide a method for preparing starch and a non-thermal-denaturation protein powder by using rice as a raw material.

### SUMMARY

In view of the problems existing in the art, the present application proposes a method for preparing starch and a non-thermal-denaturation protein powder by using rice as a raw material, which combines physical extraction with low-temperature enzymatic extraction, and achieves the objects of environmental protection during extraction, improving the quality of starch and protein and reducing the production cost.

The technical problems to be solved by the present application are as follows:
a method for preparing starch and protein powder by using rice as a raw material includes the steps of:
S1, crushing rice, adding water and performing homogenizing to prepare a slurry;
S2, removing water from the slurry, adding a specific gravity regulator into a solid phase to regulate a specific gravity of the slurry to be greater than that of protein, and less than that of starch;
S3, subjecting a mixture in the step S2 to one or more separations to obtain a liquid phase, a light phase and a heavy phase;
S4, diluting the light phase in the step S3 with water, adding a medium-temperature amylase, performing enzymolysis, and performing centrifugal separation to obtain a protein precipitate and a syrup liquid;
S5, preparing the protein powder from the protein precipitate in the step S4, and preparing a syrup from the syrup liquid;
S6, washing the heavy phase in the step S3 with water, and performing centrifugal water removal to obtain wet starch;
S7, preparing the starch from the wet starch in the step S6; and
S8, concentrating the liquid phase in the step S3 to prepare a syrup.

Further, the slurry in the step S1 is homogenized under a pressure of more than 15 MPa.

Further, the specific gravity regulator in the step S2 regulates the specific gravity of the slurry to 1.2-1.35.

Further, the specific gravity regulator adopts one or more of maltodextrin, maltose, fructose, and glucose.

Further, part of wastewater obtained after removing water in the step S2 is reused to the step S1 and the other part of the wastewater is used for washing the heavy phase; wastewater obtained after the water removal in the step S6 is mixed with the syrup liquid in the step S5 to prepare the syrup; and part of the concentrated liquid phase in the step S8 is mixed with the syrup liquid in the step S5 to prepare the syrup, and the other part of the concentrated liquid phase is directly used as the specific gravity regulator.

Still further, a method for preparing the syrup from the syrup liquid specifically includes saccharification, decolorization, ion exchange, and evaporation.

Still further, the syrup is reused to the step S2 as the specific gravity regulator.

Further, in the step S4, the light phase is diluted with water by 1-10 times; and the enzymolysis is performed at a temperature of less than 60°C.

Further, in the step S5, the protein precipitate is washed with water, separated by centrifugation, sterilized by using microwave vacuum drying equipment, and dried to obtain the protein powder; and the wet starch is dried to prepare the starch in the step S7.

Further, protein can be further purified from the light phase in the step S3 by diluting the light phase with water, regulating a specific gravity to be less than that of protein, and performing centrifugation, the heavy phase being a mixture of protein and starch.

The beneficial effects of the present application are as follows:
the present application provides a method for preparing starch and a non-thermal-denaturation protein powder by using rice as a raw material, after the rice is crushed, and pulping, homogenizing, and centrifugal water removal are performed, the specific gravity regulator is added, and then through one or more separations, a material is divided into three phases: a heavy phase, a light phase and a liquid phase, wherein the heavy phase is used for preparing starch, the light phase is used for preparing a protein powder and a syrup, and part of the liquid phase is used for the specific gravity regulator, and the other part of the liquid phase is used for preparing the syrup; wastewater generated during the preparation process is reused for the preparation of the syrup; and the syrup is used as the specific gravity regulator. The method of the present application adopts a physical method combined with a low-temperature enzymatic method to extract the starch and the protein powder while producing the syrup. The syrup can be used as the specific gravity regulator. The syrup comes from the rice, and foreign matters are not added, which ensures the safety of the product. The protein is purified, dried, and sterilized at low temperature, without denaturation. The extraction rate is high, the process is simple, the production cost is low, and the product quality is good. The produced syrup product not only provides the specific gravity regulator for the separation of the starch from the protein powder, and but also solves the problem of outlet of the syrup obtained after enzymolysis during protein purification, and also solves the problem of the treatment of a large amount of wastewater containing syrup, starch and protein generated by the washing process of starch and protein, which not only improves the yield of the product, but also reduces the difficulty of wastewater treatment.

### BRIEF DESCRIPTION OF THE DRAWINGS

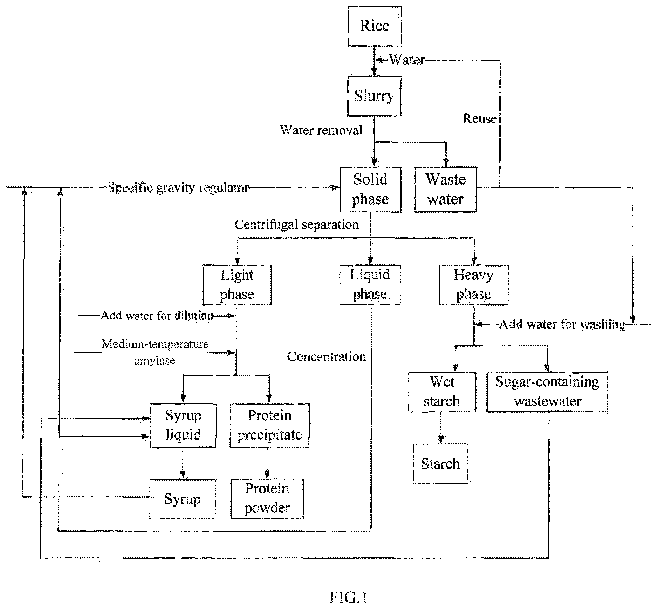

FIG. 1 is a flow chart of a process for preparing starch and a non-thermal-denaturation protein powder by using rice as a raw material according to an example of the present application.

### DETAILED DESCRIPTION OF THE EMBODIMENTS

The technical solutions in the examples of the present application will be clearly and completely described below in conjunction with the examples of the present application. Obviously, the described examples are only some of the examples of the present application, rather than all of the examples. Based on the examples in the present application, all other examples obtained by those of ordinary skill in the art without making inventive labor belong to the scope of protection of the present application.

It should be noted that in the present application, all the embodiments and preferred embodiments mentioned herein can be combined with each other to form new technical solutions unless otherwise specified. In the present application, all the technical features and preferred features mentioned herein can be combined with each other to form new technical solutions unless otherwise specified. In the present application, percentages (%) or parts refer to weight percentages or weight parts relative to the composition unless otherwise specified. In the present application, the components involved or preferred components thereof can be combined with each other to form new technical solutions unless otherwise specified. In the present application, unless otherwise specified, a numerical range "a-b" represents an abbreviated representation of any combination of real numbers between a and b, where both a and b are real numbers. For example, a numerical range of "6-22" means that all real numbers of "6-22" have been listed herein in their entirety, and "6-22" is merely an abbreviated representation of these numerical combinations. "Ranges" disclosed in the present application can be one or more lower limits and one or more upper limits, respectively in the form of a lower limit and an upper limit. In this application, unless otherwise specified, each reaction or operation step may be performed in sequence or in order. Preferably, the reaction process herein is carried out sequentially.

Unless defined otherwise, technical and scientific terms used herein have the same meanings those familiar to those skilled in the art. In addition, any method or material similar or equivalent to the contents described can also be applied in this application.

The test materials used in the following examples, unless otherwise specified, were all purchased from conventional biochemical reagent stores.

Quantitative experiments in the following examples were performed in triplicate and data is a mean of triplicate experiments or mean ± SD (Standard Deviation).

As shown in FIG. 1, a method for preparing starch and a non-thermal-denaturation protein powder by using rice as a raw material included the following steps:
S1, after the rice was cleaned and crushed, water was added, and homogenizing was performed under a pressure of more than 15 MPa to prepare a slurry with a mass concentration of 5%-40%;
S2, water was removed from the slurry, and part of wastewater obtained after water removal was reused to the step S1, the other part of the wastewater was used for washing a heavy phase; and a specific gravity regulator was added into a solid phase to regulate a specific gravity of the slurry to be greater than a specific gravity of rice protein and less than a specific gravity of rice starch, wherein preferably, the specific gravity was regulated to 1.2-1.35;
S3, a mixture in the step S2 was separated to obtain a liquid phase, a light phase and a heavy phase, wherein the separation is performed for one or more times;
S4, the light phase in the step S3 was diluted with water by 1-10 times, a medium-temperature amylase was added, enzymolysis was performed at a temperature of less than 60°C, and centrifugal separation was performed to obtain a protein precipitate and a syrup liquid, wherein the enzymolysis included hydrolyzing residual starch in the protein to be converted into a syrup which is dissolved in water;
S5, the protein precipitate in the step S4 was washed with water, separated by centrifugation, sterilized by using microwave vacuum drying equipment, and dried to prepare protein powder; and a syrup was prepared from the syrup liquid, wherein a method for preparing the syrup from the syrup liquid specifically included saccharification, decolorization, ion exchange, and evaporation;
S6, the heavy phase in the step S3 was washed with water, and centrifugal water removal was performed to obtain wet starch; and wastewater obtained after the water removal was mixed with the syrup liquid in the step S5 to prepare a syrup;
S7, the wet starch in the step S6 was dried to prepare starch; and
S8, part of the liquid phase in the step S3 was mixed with the syrup liquid prepared in the step S5 for the specific gravity regulator, and the other part of the liquid phase was used for preparing a syrup.

In some examples of the present application, the specific gravity regulator adopted one or more of maltodextrin, maltose, fructose, and glucose.

In some examples of the present application, the syrup prepared in the step S5 was reused to the step S2 as the specific gravity regulator.

In some examples of the present application, protein can be further purified from the light phase in the step S3 by diluting the light phase with water, regulating a specific gravity to less than that of rice protein, and performing centrifugation, the heavy phase being a mixture of protein and starch; and the light phase being a mixture having a specific gravity less than that of the protein, including syrup and other impurities.

In some examples of the present application, a homogenizer in the step S1 adopted a high-pressure homogenizer, the homogenization pressure was more than 15 MPa, and the temperature was controlled not to exceed 55°C; rice milk components were crushed by using the high-pressure homogenizer and then physical centrifugation was performed by using a centrifuge to separate protein and starch. The production technique is a physical technique that does not change the nature of the protein, which is advantageous to maintain the properties and mouthfeel of the protein, and to avoid starch gelatinization and protein denaturation. The water removal employed a centrifuge or other solid-liquid separation equipment; the mixing employed a colloid mill in the step S2; and in the step S3, the centrifuging is performed by using a two-phase horizontal screw centrifuge, a three-phase horizontal screw centrifuge, or a disc centrifuge.

In some examples of the present application, all of non-starch and non-protein components produced by the production process entered the syrup production process in the step S5.

On the basis of the above examples, the rice includes polished long-grained non-glutinous rice, polished round-grained rice, and glutinous rice.

On the basis of the above examples, other fields of application using specific gravity regulators to solve the problem of the separation of solids of different densities are also within the scope of protection of this patent.

### Example 1

1000 kg of rice was cleaned and crushed to 60-mesh, then 2000 kg of water was added, pulp grinding was performed, and uniform stirring was performed. Treatment was performed by using a high-pressure homogenizer under a homogenization pressure of 20 KPa at a temperature controlled to be less than 50°C. Next, centrifugal separation was performed by using a horizontal screw centrifuge to obtain wastewater and a solid precipitate, and the wastewater can be recycled. 2000 kg of 75% rice maltose syrup was added into about 1800 kg of the solid, uniform stirring was performed, then water was added, and a specific gravity was regulated to 1.25, and uniform stirring was performed. Then, three-phase separation was performed by using a three-phase horizontal screw centrifuge to obtain a light phase, a liquid phase, and a heavy phase. The light phase was a solid suspension, and was a mixture of protein and starch, about 700 kg, and 1400 kg of water was added, uniform stirring was performed, then heating was performed to 55°C, 0.3 kg of a medium-temperature amylase was added, stirring was performed for 2 h, then separation was performed with a disc centrifuge to obtain a protein precipitate and a syrup liquid. The obtained protein precipitate was washed with water, separated by disc centrifugation, and then subjected to microwave vacuum drying to obtain rice protein powder, wherein the prepared rice protein powder had no thermal deformation and had a purity of more than 80%. The syrup liquid produced after the separation was heated to 60°C, 0.1 kg of a saccharifying enzyme was added, and saccharification was performed for 10 h to obtain a maltose syrup, then heating was performed to 80°C, 0.2% activated carbon was added, the obtained mixture was left with stirring for 30 min, and filtered, the activated carbon was removed, followed by entering an ion exchange system, desalting and decolorizing, and then, the syrup was concentrated to a solid content of 75% by using a four-effect evaporator. The liquid phase obtained after the three-phase separation was diluted a rice syrup, part of the liquid phase was reused and the other part of the liquid phase entered a rice syrup production process. A solid phase obtained after the three-phase separation was rice starch, had a weight of about 1200 kg, was washed with 3000 kg of water, and was then separated by a horizontal screw centrifuge to obtain solid phase starch and wastewater, and then washing was performed with water, and separation was performed, and the above operations were repeated to be performed for 4 times to obtain rice starch meeting the requirements, and the rice starch was then subjected to air flow drying to obtain rice starch with a water content of less than 14% and a purity of more than 99%. Wastewater produced in the starch washing process can be used for the above washing. Concentrated wastewater contained the syrup liquid and entered the rice syrup production process.

### Example 2

1500 kg of rice was cleaned and crushed to 50-mesh, then 3000 kg of water was added, pulp grinding was performed, and uniform stirring was performed. Treatment was performed by using a high-pressure homogenizer under a homogenization pressure of 25 KPa at a temperature controlled to be less than 50°C. Next, centrifugal separation was performed by using a horizontal screw centrifuge to obtain wastewater and a solid precipitate, and the wastewater can be recycled. 2500 kg of 80% rice maltose syrup was added into about 2700 kg of the solid, uniform stirring was performed, then water was added, and a specific gravity was regulated to 1.27, and uniform stirring was performed. Then, two-phase separation was performed by using a horizontal screw centrifuge to obtain a light phase and a heavy phase. The light phase was a solid suspension, and was a mixture of protein, starch and syrup, about 2500 kg, and was diluted with water to a specific gravity of 1.2, uniform stirring was performed, and then centrifugal separation was performed by using a horizontal screw centrifuge, wherein the obtained precipitate was a mixture of protein and starch, and a light phase was a thin syrup, part of the thin syrup was reused, and the other part of the thin syrup entered a rice syrup production process to be processed. 2000 kg of washing wastewater produced in the production process was added into about 1000 kg of the obtained precipitate of starch and protein, heating was performed to 58°C, then 0.5 kg of a medium-temperature amylase was added, stirring was performed for 2 h, and then separation was performed by a disc centrifuge to obtain a protein precipitate and a syrup liquid. The obtained protein precipitate was washed with water, separated by disc centrifugation, and then subjected to microwave vacuum drying to obtain rice protein powder, wherein the prepared rice protein powder had no thermal deformation and had a purity of more than 80%. The syrup liquid produced after the separation was heated to 60°C, 0.15 kg of a saccharifying enzyme was added, and saccharification was performed for 10 h to obtain a maltose syrup, then heating was performed to 80°C, 0.3% activated carbon was added, the obtained mixture was left with stirring for 30 min, and filtered, the activated carbon was removed, followed by entering an ion exchange system, desalting and decolorizing, and then, the syrup was concentrated to a solid content of 80% by using a four-effect evaporator. A solid phase obtained after the two-phase separation was rice starch, had a weight of about 1800 kg, was washed with 4000 kg of water, and was then separated by a horizontal screw centrifuge to obtain solid phase starch and wastewater, and then washing was performed with water, and separation was performed, and the above operations were repeated to be performed for 5 times to obtain rice starch meeting the requirements, and the rice starch was then subjected to air flow drying to obtain rice starch with a water content of less than 14% and a purity of more than 99%. Wastewater produced in the starch washing process can be used for the above washing. Concentrated wastewater contained the syrup liquid and entered the rice syrup production process.

### Example 3

2000 kg of rice was cleaned and crushed to 40-mesh, then 4000 kg of water was added, pulp grinding was performed, and uniform stirring was performed. Treatment was performed by using a high-pressure homogenizer under a homogenization pressure of 18 KPa at a temperature controlled to be less than 55°C. Next, centrifugal separation was performed by using a horizontal screw centrifuge to obtain wastewater and a solid precipitate, and the wastewater can be recycled. 4000 kg of 75% rice fructose-glucose syrup (the fructose content being 42%) was added into about 3600 kg of the solid, uniform stirring was performed, then water was added, and a specific gravity was regulated to 1.29, and uniform stirring was performed. Then, three-phase separation was performed by using a three-phase horizontal screw centrifuge to obtain a light phase, a liquid phase, and a heavy phase. The light phase was a solid suspension, and was a mixture of protein and starch, about 1400 kg, and 2800 kg of water was added, uniform stirring was performed, then heating was performed to 57°C, 0.6 kg of a medium-temperature amylase was added, stirring was performed for 2 h, then separation was performed with a disc centrifuge to obtain a protein precipitate and a syrup liquid. The obtained protein precipitate was washed with water, separated by disc centrifugation, and then subjected to microwave vacuum drying to obtain rice protein powder, wherein the prepared rice protein powder had no thermal deformation and had a purity of more than 80%. The syrup liquid produced after the separation was heated to 60°C, 0.1 kg of a saccharifying enzyme was added, and saccharification was performed for 36 h to obtain a glucose syrup, then heating was performed to 80°C, 0.2% activated carbon was added, the obtained mixture was left with stirring for 30 min, and filtered, the activated carbon was removed, followed by entering an ion exchange system, desalting and decolorizing, and then, an isomerase was added to convert glucose to a fructose-glucose mixture (the fructose content being 42%), then the above decolorizing and ion exchange process were repeated, and finally part of the syrup was concentrated to a solid content of 71% by using a four-effect evaporator for commercial sale, and the other part of the syrup was concentrated to a solid content of 75% by using the four-effect evaporator for a specific gravity regulator. The liquid phase obtained after the three-phase separation was diluted a rice syrup, part of the liquid phase was reused and the other part of the liquid phase entered a rice syrup production process. A solid phase obtained after the three-phase separation was rice starch, had a weight of about 2400 kg, was washed with 6000 kg of water, and was then separated by a horizontal screw centrifuge to obtain solid phase starch and wastewater, and then washing was performed with water, and separation was performed, and the above operations were repeated to be performed for 4 times to obtain rice starch meeting the requirements, and the rice starch was then subjected to air flow drying to obtain rice starch with a water content of less than 14% and a purity of more than 99%. Wastewater produced in the starch washing process can be used for the above washing. Concentrated wastewater contained the syrup liquid and entered the rice syrup production process.

The above are only the preferred embodiments of the present application. It should be pointed out that for those of ordinary skill in the art, several improvements and replacements can be made without departing from the technical principle of the present application, and these improvements and replacements should also be regarded as the protection scope of the present application.

## Claims

1. A method for preparing starch and a non-thermal-denaturation protein powder by using rice as a raw material, comprising the steps of:
S1, crushing the rice, adding water and performing homogenizing to prepare a slurry;
S2, removing water from the slurry, adding a specific gravity regulator into a solid phase to regulate a specific gravity of the slurry to be greater than that of protein, and less than that of starch;
S3, subjecting a mixture in the step S2 to one or more separations to obtain a liquid phase, a light phase and a heavy phase;
S4, diluting the light phase in the step S3 with water, adding a medium-temperature amylase, performing enzymolysis, and performing centrifugal separation to obtain a protein precipitate and a syrup liquid;
S5, preparing a protein powder from the protein precipitate in the step S4, and preparing a syrup from the syrup liquid;
S6, washing the heavy phase in the step S3 with water, and performing centrifugal water removal to obtain wet starch;
S7, preparing starch from the wet starch in the step S6; and
S8, concentrating the liquid phase in the step S3 to prepare a syrup.

2. The method for preparing starch and a non-thermal-denaturation protein powder by using rice as a raw material according to claim 1, wherein the slurry in the step S1 is homogenized under a pressure of more than 15 MPa.

3. The method for preparing starch and a non-thermal-denaturation protein powder by using rice as a raw material according to claim 1, wherein the specific gravity regulator in the step S2 regulates the specific gravity of the slurry to 1.2-1.35.

4. The method for preparing starch and a non-thermal-denaturation protein powder by using rice as a raw material according to claim 1, wherein the specific gravity regulator adopts one or more of maltodextrin, maltose, fructose, and glucose.

5. The method for preparing starch and a non-thermal-denaturation protein powder by using rice as a raw material according to claim 1, wherein part of wastewater obtained after removing water in the step S2 is reused to the step S1 and the other part of the wastewater is used for washing the heavy phase; wastewater obtained after the water removal in the step S6 is mixed with the syrup liquid in the step S5 to prepare the syrup; and part of the concentrated liquid phase in the step S8 is mixed with the syrup liquid in the step S5 to prepare the syrup, and the other part of the concentrated liquid phase is directly used as the specific gravity regulator.

6. The method for preparing starch and a non-thermal-denaturation protein powder by using rice as a raw material according to claim 5, wherein a method for preparing the syrup from the syrup liquid specifically comprises saccharification, decolorization, ion exchange, and evaporation.

7. The method for preparing starch and a non-thermal-denaturation protein powder by using rice as a raw material according to claim 1 or 5, wherein the syrup is reused to the step S2 as the specific gravity regulator.

8. The method for preparing starch and a non-thermal-denaturation protein powder by using rice as a raw material according to claim 1, wherein in the step S4, the light phase is diluted with water by 1-10 times; and the enzymolysis is performed at a temperature of less than 60°C.

9. The method for preparing starch and a non-thermal-denaturation protein powder by using rice as a raw material according to claim 1, wherein in the step S5, the protein precipitate is washed with water, separated by centrifugation, sterilized by using microwave vacuum drying equipment, and dried to obtain the protein powder; and the wet starch is dried to prepare the starch in the step S7.

10. The method for preparing starch and a non-thermal-denaturation protein powder by using rice as a raw material according to claim 1, wherein protein can be further purified from the light phase in the step S3 by diluting the light phase with water, regulating a specific gravity to be less than that of protein, and performing centrifugation, the heavy phase being a mixture of protein and starch.
